# EUROPEAN PATENT APPLICATION

(11) **EP 4 613 742 A1**
(43) Date of publication of application: **10.09.2025**
(21) Application number: 24162085.5
(22) Date of filing: 07.03.2024
(51) Int. Cl.: C07D 319/12

(54) **A METHOD AND PLANT FOR PREPARING A CRUDE LACTIDE COMPOSITION USING A LACTIDE SYNTHESIS REACTOR COMPRISING A DISTILLATION COLUMN AND A LOOP REACTOR**

(71) Applicant: Sulzer Management AG, 8401 Winterthur (CH)
(72) Inventor: The designation of the inventor has not yet been filed
(74) Representative: IPS Irsch AG

(57) **Abstract**

The present invention relates to a method for preparing a crude lactide composition containing L-lactide and meso-lactide comprising the steps of subjecting a lactic acid composition to a prepolymerization reaction in a prepolymerization reactor so as to obtain a prepolymerized composition and then subjecting the prepolymerized composition to a depolymerization reaction in a lactide synthesis reactor, which comprises a distillation column and a loop reactor being connected therewith, so as to obtain the crude lactide composition, wherein the prepolymerized composition is fed into the distillation column, from which an overhead composition, a side composition as the crude lactide composition and a bottom composition are withdrawn, wherein the bottom composition is led into the loop reactor at a first position, wherein reaction product is withdrawn from the loop reactor at a second position and is led into the distillation column.

## Description

The present invention relates to a method and to a plant for producing and preferably for continuously producing a crude lactide composition from lactic acid.

Polymers of lactic acid are of particular interest because they can be obtained from renewable resources and are mostly compostable and/or biodegradable. Moreover, the technological and physiochemical properties of these polymers come quite close to the properties of those polymers derived from fossil-based resources, which explains why these polymers are regarded as highly promising substitutes for the latter. Furthermore, polymers of lactic acid have a wide range of applications. For instance, polylactic acid is used in the biomedical field in chirurgical implants, in films, such as e.g. in packagings, in fibers, such as e.g. for garments, in hygienic articles, in carpets and in disposable plastic products, such as e.g. disposable cutlery or containers. In addition, polylactic acid has found wide application in composite materials, such as in fiber-reinforced plastics.

Generally, two alternative principal methods are known for synthesizing polylactic acid. The first principal method is the direct polycondensation of lactic acid to polylactic acid. However, this principal method only leads to low molecular weight polymers and is thus limited to specific polymers.

The second principal method known for synthesizing polylactic acid is the ring-opening-polymerization of lactide, which is the cyclic diester of lactic acid. This is the preferred method nowadays for the industrial production of polylactic acid. In principle, the lactide may be produced by condensation of two lactic acid molecules, which is, however, a quite inefficient method. Therefore, lactide is industrially predominantly produced by first prepolymerizing lactic acid and then subjecting the oligomer or prepolymer, respectively, to a depolymerization reaction. For instance, the lactide is prepared by fermentation of carbohydrates from biomass, such as starch, sugar or corn resulting in lactic acid, by then prepolymerizing the lactic acid and by afterwards subjecting the prepolymers to the depolymerization reaction. The depolymerization reaction of the lactic acid prepolymer to lactide is an equilibrium reaction. In order to push the reaction towards the lactide, lactide must be withdrawn from the system. In a continuous depolymerization, in which the lactic acid prepolymer is fed continuously to a lactide synthesis reactor, crude lactide is therefore evaporated under vacuum. The lactide synthesis reactor may be a stirred tank reactor, a stirred tank reactor with an evaporator on its top, a wiped film evaporator, a thin film evaporator or a distillation column. The lactide synthesis reactor produces a crude lactide composition that contains the lactide and lactic acid, lactic acid prepolymers and other impurities. This crude lactide composition is usually purified and the purified lactide is then polymerized in the presence of a catalyst and optionally an initiator so as to form a crude polylactic acid composition, such as a crude high molecular weight polylactic acid composition. After the polymerization, the unreacted lactide has to be removed from the crude polylactic acid composition to a final concentration of less than at least 0.5 % by weight, in order to obtain a product of marketable quality. The removal of unreacted lactide as well as of other low-boiling components of the crude polylactic acid composition is usually done by devolatilization. More specifically, the crude polylactic acid composition is subjected to one or more devolatilization steps, which are conducted at an elevated temperature and at a reduced pressure. During the devolatilization, the unreacted lactide as well as other low-boiling and close-boiling components of lactide, components formed as side-products with the polymerization in the polylactic acid reactor are removed as vapor fraction from the polylactic acid so as to obtain a vapor composition including the unreacted lactide and a liquid fraction including purified polylactic acid melt. The vapor composition including the unreacted lactide is then condensed and may be recycled into the polymerization reaction.

Lactic acid is chiral and exists in the form of enantiomeric L-lactic acid and D-lactic acid, which are also called S-lactic acid and R-lactic acid, respectively. Therefore, lactide, i.e. the dimer of lactic acid, exists in the form of three stereoisomers, namely as L-lactide (or S,S-lactide, respectively), as D-lactide (or R,R-lactide, respectively) and as meso-lactide (or S,R-lactide, respectively). The properties of the polylactic acid, such as the processing properties, crystallization properties and degradation behavior of polylactic acid, depend on the structure and composition of the polymer chains, in particular on the ratio of the L- to the D-stereoisomer of lactic acid. The stereochemical structure of polylactic acid can be modified by copolymerization of mixtures of L-lactide, meso-lactide and D-lactide resulting in high molecular weight amorphous or semicrystalline polymers with a melting point in the range from 130 to 185°C. Furthermore, homopolymers may be produced consisting only of L-lactic acid or of D-lactic acid monomers. For instance, isotactic polylactic acid homopolymer, which comprises only L-lactic acid monomer, is a semicrystalline material with a highest melting point, whereas polylactic acid copolymers comprising L-lactic acid and D-lactic acid monomers with a comparable high D-stereoisomer content exhibit lower melting points and slower crystallization behavior. Therefore, processes are desirable, in which polylactic acid with a predetermined stereoisomeric structure may be easily and reliably produced from lactide.

As set out above, the lactide synthesis reactor, in which the depolymerization of the lactic acid prepolymer to lactide is performed, may be a stirred tank reactor, a stirred tank reactor with an evaporator on its top, a wiped film evaporator, a thin film evaporator or a distillation column, in which the synthesis mainly takes place in the sump of the distillation column, but also in other sections where lactide composition is not in equilibrium with lactic acid prepolymers. However, all these reactor types lead to a comparable low lactide yield. One of the reasons therefore is that it is difficult or impossible to adjust in all reactor compartments of these reactors a homogenous reaction mixture, a sufficient high and homogeneous catalyst concentration, an uniform temperature and an uniform pressure. This does not only lead to a comparable low lactide yield, but also to a comparable high catalyst consumption as well as a comparable high racemization degree, thus leading undesirably to a high D-lactide content in the lactide composition.

In view of this, the object underlying the present invention is to provide a method and a plant for producing a crude lactide composition containing L-lactide and meso-lactide, which is characterized by a high lactide yield, by a low catalyst consumption as well as by a low racemization degree.

In accordance with the present invention this object is satisfied by providing a method for preparing a crude lactide composition containing L-lactide and meso-lactide comprising the steps of subjecting a lactic acid composition to a prepolymerization reaction in a prepolymerization reactor so as to obtain a prepolymerized composition and then subjecting the prepolymerized composition to a depolymerization reaction in a lactide synthesis reactor, which comprises a distillation column and a loop reactor being connected therewith, so as to obtain the crude lactide composition, wherein the prepolymerized composition is fed into the distillation column, from which an overhead composition, a side composition as the crude lactide composition and a bottom composition are withdrawn, wherein the bottom composition is led into the loop reactor at a first position, wherein reaction product is withdrawn from the loop reactor at a second position and is led into the distillation column.

This solution bases on the finding that by performing the lactide synthesis reaction or depolymerization reaction of the prepolymerized composition to the crude lactide composition, respectively, in the lactide synthesis reactor, which comprises a distillation column and a loop reactor being connected therewith, - wherein the prepolymerized composition is fed into the distillation column, from which an overhead composition, a side composition as the crude lactide composition and a bottom composition are withdrawn, wherein the bottom composition is led into the loop reactor at a first position, wherein reaction product is withdrawn from the loop reactor at a second position and is led into the distillation column - a crude lactide composition is obtained with a high lactide yield, wherein the method is further characterized by a low catalyst consumption as well as a low racemization degree and thus by a comparable low D-lactide content in the crude lactide composition. Without being wished to be bound to any theory, it is considered that these advantages are achieved among others on account of the fact that thereby the depolymerization of the polylactic prepolymer to lactide does at least predominantly, if not exclusively occur in the loop reactor, which allows to adjust a homogenous reaction mixture, a sufficiently high and homogeneous catalyst concentration, an uniform temperature and an uniform pressure and thus to perform the depolymerization of the polylactic prepolymer to lactide and an optimal homogenous catalyst concentration, temperature and pressure. The obtained reaction product, which is withdrawn from the loop reactor at the second position and is led into the distillation column, is then evaporated in the distillation column so that lights, such as lactic acid and water, are removed from the distillation column as overhead composition, thus shifting the reaction to towards the production of lactide, whereas the crude lactide composition is withdrawn from the distillation column and thus from the lactide synthesis reactor as side composition.

The distillation column of the lactide synthesis reactor is subsequently also denoted as first distillation column, in order to distinguish it from other distillation columns, which may be comprised and which are described further below.

The present invention is not particularly limited concerning the content of lactic acid in the lactic acid composition being used as starting composition in the prepolymerization reaction. Good results are, however, obtained, when the lactic acid composition used as starting composition in the prepolymerization reaction contains, based on 100% by weight of the lactic acid composition, at least 80% by weight and preferably at least 90% by weight of lactic acid.

Also concerning the ratio of the two stereoisomers, i.e. L-lactic acid and D-lactic acid, the present invention is not particularly restricted. For instance, the lactic acid composition used as starting composition in the prepolymerization reaction contains, based on 100% by weight of the lactic acid being contained in the lactic acid composition, at least 90% by weight and more preferably 97% by weight of L-lactic acid and remainder to 100% by weight of D-lactic acid.

In a further development of the idea of the present invention, it is suggested that the prepolymerization reaction is performed at a temperature of up to 190°C and more preferably at a temperature between 150 and 190°C.

In accordance with a further preferred embodiment of the present invention, the prepolymerization reaction is performed in up to three subsequent reactors at a pressure of 1 to 35 kPa and preferably of 2 to 31 kPa.

By appropriately selecting the temperature and pressure, the prepolymerization reaction may be controlled so that the prepolymerized composition obtained in the prepolymerization reaction contains lactic acid prepolymer with an average polymerization degree of 7 to 20.

In accordance with the present invention, the prepolymerized composition is fed into the (first) distillation column of the lactide synthesis reactor. For instance, the prepolymerized composition is fed into the lower part or bottom part, respectively, of the (first) distillation column. For example, the prepolymerized composition is fed at a position being located, seen from the bottom to the top of the (first) distillation column, at 0 to 50%, preferably 1 to 40% and more preferably 10 to 30% of the height of the (first) distillation column.

In order to increase the reaction speed and yield, any catalyst usually used for depolymerizing polylactic acid prepolymer into lactide may be used in the method. The catalyst is preferably (only) added to the loop reactor and thus contained (only) in the loop reactor of the lactide synthesis reactor. Suitable examples for catalysts are catalysts being selected from the group consisting of tin oxide (SnO), tin chloride (SnCl₂), Sn(octanoate)₂, tin (II) octoate, antimony oxide (Sb₂O₃), sulfuric acid (H₂SO₄), dibutyltin oxide (Bu₂SnO,) zinc oxide and arbitrary combinations of two or more of the aforementioned catalysts.

Good results in terms of lactide yield are in particular obtained, when the loop rector is heated to a temperature of 190 to 240°C and preferably of 195 to 197°C. For this purpose, the loop reactor may comprise one or more heat exchangers, in which the mixture contained in the loop reactor is heated to the aforementioned temperature.

In order to adjust a particular homogenous reaction mixture composition, sufficient and homogenous catalyst concentration, uniform temperature and uniform pressure in the loop reactor of the lactide synthesis reactor, it is proposed in a further development of the idea of the present invention that the loop rector comprises at least one mixer. The at least one mixer may be in principle a dynamic mixer or a static mixer, wherein a static mixer is preferred. Good results are in particular obtained, when the at least one static mixer is selected from the group consisting of x-type static mixers, spiral/helical-type static mixers, quattro-type static mixers, baffle plate-type static mixers, turbulator strips-type static mixers and any combination of two or more of the above mentioned mixer types. Preferably, the at least one mixer is a heatable static mixer, thus allowing to mix and simultaneously feed the required heat into the reaction mixture.

In accordance with a further preferred embodiment of the present invention, purge is continuously withdrawn from the loop reactor. This allows to reliably avoid the accumulation of any side-products within the loop reactor.

Good results are in particular obtained, when the residence time in the loop reactor is below 8 hours and more preferably below 6 hours.

In order to improve the mass and heat transfer and thereby the separation efficiency within the (first) distillation column, the (first) distillation column preferably comprises at least one, more preferably one to ten, yet more preferably two to five, still more preferably two to four and most preferably three beds of an internal being selected from structured packings, random packings, plates, pipe bundles and arbitrary combinations of two or more thereof.

Furthermore, it is preferred that the (first) distillation column is operated at a temperature of below 230°C and at a pressure of below 1 kPa. More preferably, the (first) distillation column is operated at a temperature of below 210°C and at a pressure of below 0.5 kPa.

In accordance with the present invention, during the method an overhead composition, a side composition and a bottom composition are withdrawn from the (first) distillation column. While the overhead composition contains lights, such as lactic acid and water, the side composition is the crude lactide composition and the bottom composition contains lactic acid prepolymer, which is led into the loop reactor, where it is converted into lactide. Preferably, the side composition or crude lactide composition, respectively, is withdrawn from the (first) distillation column at a position being located above the position, at which the prepolymerized composition is fed into the (first) distillation column, and preferably at a position being located, seen from the bottom to the top of the (first) distillation column, at 20 to 60%, preferably 25 to 50% and more preferably 25 to 40% of the height of the (first) distillation column.

In accordance with a particular preferred embodiment of the present invention, the (first) distillation column comprises at least two and preferably at least three beds of an internal being selected from structured packings, random packings, plates, pipe bundles and arbitrary combinations of two or more thereof, wherein the side composition or crude lactide composition, respectively, is withdrawn from the distillation column at a position being located between two adjacent beds of an internal and preferably above two beds of an internal.

In order to convert at least essentially all of the lactic acid to lactide so as to increase the yield of lactide based on the lactic acid used in the method as starting material, it is suggested in a further development of the idea of the present invention that at least a portion of the lactic acid contained in the overhead composition being obtained in the (first) distillation column is recycled into the prepolymerization reactor. Accordingly, it is preferred that the overhead composition is led into a condenser and is condensed therein, wherein a portion of the so obtained condensate is withdrawn and preferably recycled into the prepolymerization reactor, whereas the remaining portion thereof is recycled into the distillation column.

As set out above, the method in accordance with the present invention is characterized by a high lactide yield. For instance, the crude lactide composition being withdrawn from the (first) distillation column comprises, based on 100% by weight of the crude lactide composition, at least 85% by weight and more preferably at least 92% by weight of lactide.

In accordance with a further aspect, the present invention relates to a method for preparing a purified L-lactide composition and optionally also a purified meso-lactide composition, which comprises the following steps:
a) preparing a crude lactide composition containing L-lactide and meso-lactide with the aforementioned method,
b) feeding the crude lactide composition obtained in step a) into a second distillation column and distilling it therein into an overhead composition, into a bottom composition and into a side composition and
c) feeding the side composition obtained in step b) into a third distillation column and distilling it therein into an overhead composition being a meso-lactide enriched composition, a side composition being a L-lactide enriched composition and a bottom composition.

In order to obtain a meso-lactide enriched composition as well as a L-lactide enriched composition being pure enough to be used in a polymerization reaction, it is proposed in a further development of the idea of the present invention to purify both compositions, i.e. the meso-lactide enriched composition as well as the L-lactide enriched composition. Preferably, the L-lactide enriched composition obtained in step c) is purified by subjecting it to at least one desublimation step and/or to at least one falling film crystallization step.

In addition, it is preferred that the meso-lactide enriched composition obtained in step c) is purified by subjecting it to at least one desublimation step and/or to at least one static crystallization step.

In order to improve the yield, it is moreover preferred that the overhead composition obtained in the second distillation step b) is recycled to the prepolymerization reaction, i.e. into the prepolymerization reactor, whereas the bottom composition obtained in the second distillation step b) is preferably recycled to the depolymerization step, i.e. into the lactide synthesis reactor, and the bottom fraction obtained in the third distillation step c) is recycled to the second distillation step b), i.e. into the second distillation column.

In accordance with a further aspect, the present invention relates to a method for producing polylactic acid, in which the purified L-lactide composition being obtained with the aforementioned method and/or the purified meso-lactide composition being obtained with the aforementioned method is polymerized in a polymerization reactor. The obtained reaction mixture or crude polylactic acid composition, respectively, may then be devolatilized in one or more subsequent devolatilization steps, such as in two or three subsequent devolatilization steps, in order to remove lights, such as in particular unreacted lactide as well as of other low-boiling components of the crude polylactic acid composition. More specifically, the crude polylactic acid composition is preferably subjected to one or more devolatilization steps, which are conducted at an elevated temperature and at a reduced pressure. During the devolatilization, the unreacted lactide as well as other low-boiling and close-boiling components having been formed during the polymerization reaction as side-products are removed as vapor fraction from the polylactic acid so as to obtain a vapor composition including the unreacted lactide and a liquid fraction including purified polylactic acid melt. The vapor composition including the unreacted lactide is then condensed and may be recycled into the polymerization reaction, whereas the purified polylactic acid may or may not be further purified, for instance by subjecting it to one or more crystallization steps.

In accordance with a further aspect, the present invention relates to a plant for preparing a crude lactide composition containing L-lactide and meso-lactide, which comprises:
i) at least one prepolymerization reactor comprising an inlet and an outlet and
ii) a lactide synthesis reactor comprising a distillation column and a loop reactor being connected therewith, wherein the distillation column comprises an inlet being connected with the outlet of the prepolymerization reactor, an overhead outlet, a side outlet and a bottom outlet, wherein the bottom outlet is connected with an inlet being arranged at a first position of the loop reactor, and wherein the loop reactor comprises an outlet being arranged at a second position and being connected with the distillation column.

Again, the distillation column of the lactide synthesis reactor is subsequently also denoted as first distillation column, in order to distinguish it from other distillation columns, which may be comprised in the plant and which are described further below.

Preferably, the prepolymerization reactor comprises one to three, such as two or three, subsequent prepolymerization reactors.

In a further development of the idea of the present invention, it is proposed that the inlet of the (first) distillation column is arranged at a position being located, seen from the bottom to the top of the (first) distillation column, at 0 to 50%, preferably 1 to 40% and more preferably 10 to 30% of the height of the (first) distillation column.

Moreover, it is preferred that the loop of the lactide synthesis reactor further comprises an inlet for catalyst and an outlet for purge.

In accordance with a particular preferred embodiment of the present invention, the loop rector of the lactide synthesis reactor further comprises at least one heat exchanger, in order to allow to easily provide the heat into the loop reactor, which is required to maintain the optimal temperature in the loop reactor.

In order to adjust a particular homogenous reaction mixture composition, a sufficiently high and homogenous catalyst concentration, an uniform temperature and an uniform pressure in the loop reactor of the lactide synthesis reactor, it is proposed in a further development of the idea of the present invention that the loop rector comprises at least one mixer. The at least one mixer may be in principle a dynamic mixer or a static mixer, wherein a static mixer is preferred. Good results are in particular obtained, when the at least one static mixer is selected from the group consisting of x-type static mixers, spiral/helical-type static mixers, quattro-type static mixers, baffle plate-type static mixers, turbulator strips-type static mixers and any combination of two or more of the above mentioned mixer types.

Particularly preferably, the at least one mixer is a heatable static mixer, thus allowing to mix and simultaneously feed the required heat into the reaction mixture. In this embodiment, a heat exchanger is not necessary, because the heatable static mixer functions as heat exchanger in combination with functioning as static mixer.

In order to improve the mass and heat transfer and thereby the separation efficiency within the (first) distillation column, the (first) distillation column preferably comprises at least one bed of an internal, which is preferably selected from structured packings, random packings, plates, pipe bundles and arbitrary combinations of two or more thereof. Good results are in particular obtained, when the (first) distillation column comprises one to ten, more preferably two to five, still more preferably two to four and most preferably three beds of an internal being selected from structured packings, random packings, plates, pipe bundles and arbitrary combinations of two or more thereof.

The present invention is not particularly restricted concerning the position, at which the crude lactide composition is withdrawn from the (first) distillation column via a side outlet. For instance, the side outlet of the distillation column for withdrawing the crude lactide composition is arranged at a position being located, seen from the bottom to the top of the distillation column, at 20 to 60%, preferably 25 to 50% and more preferably 25 to 40% of the height of the (first) distillation column.

In accordance with a further aspect, the present invention is related to a plant for preparing a purified L-lactide composition and optionally also a purified meso-lactide composition, which comprises:
a) the aforementioned plant for preparing a crude lactide composition containing L-lactide and meso-lactide,
b) a second distillation column comprising an inlet for crude composition being connected with the side outlet of the distillation column of the lactide synthesis reactor, an overhead outlet, a bottom outlet and a side outlet, and
ii) a third distillation column comprising an inlet being connected with the side outlet of the second distillation column, wherein the third distillation column further comprises an overhead outlet, a bottom outlet and a side outlet.

In accordance with a further preferred embodiment of the present invention, the plant further comprises a recycle line connecting the overhead outlet of the second distillation column with an inlet of the prepolymerization reactor, a recycle line connecting the bottom outlet of the second distillation column with an inlet of the distillation column of the lactide synthesis reactor, and a recycle line connecting the bottom outlet of the third distillation column with an inlet of the second distillation column.

In order to obtain a meso-lactide enriched composition as well as a L-lactide enriched composition being pure enough to be used in a polymerization reaction, it is proposed in a further development of the idea of the present invention to further both compositions, i.e. the meso-lactide enriched composition as well as the L-lactide enriched composition. In view thereof, the plant preferably further comprises at least one desublimation reactor and/or to at least one static crystallizer being connected with the side outlet of the third distillation column.

Likewise thereto, it is preferred that the plant further comprises at least one desublimation reactor and/or to at least one static crystallizer being connected with the overhead outlet of the third distillation column.

In accordance with a further aspect, the present invention relates to a plant for producing polylactic acid, which comprises the aforementioned plant, which further comprises at least one desublimation reactor and/or at least one static crystallizer being connected with the side outlet of the third distillation column, and which further comprises at least one polymerization reactor, which comprises an inlet being connected with an outlet of the at least one desublimation reactor and/or at least one static crystallizer.

Subsequently, the present invention is described by means of illustrative, but not limiting figures, in which:
- Fig. 1: shows a schematic view of a plant for producing polylactic acid comprising a lactide synthesis reactor in accordance with one embodiment of the present invention.
- Fig. 2: shows a detailed schematic view of the lactide synthesis reactor of the plant shown in figure 1.

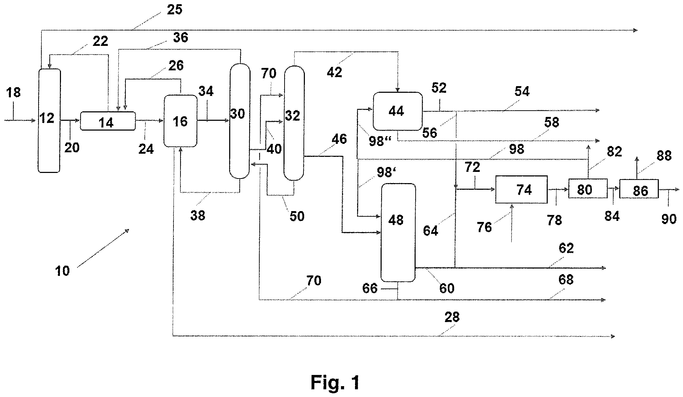

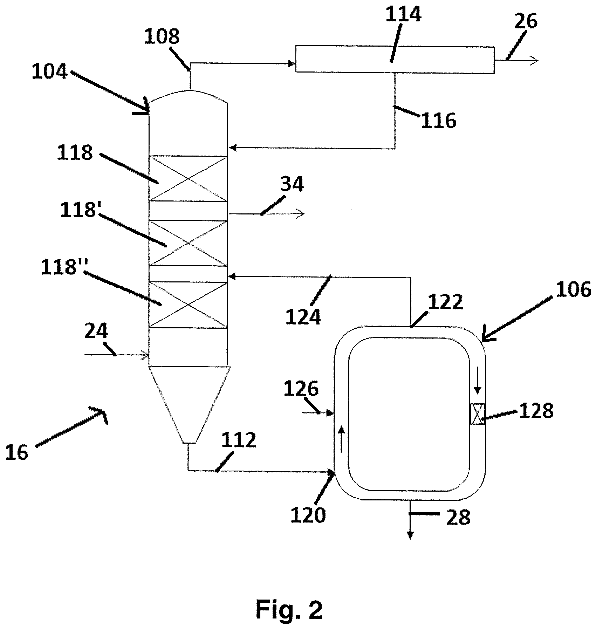

The plant 10 shown in figure 1 comprises, in series, a dewatering unit 12, a prepolymerization reactor 14 and a lactide synthesis reactor 16, wherein the dewatering unit 12 comprises an inlet line 18 for lactic acid. As in further detail described below with regard to figure 2, the lactide synthesis reactor 16 comprises a first distillation column 104 and a loop reactor 106 being connected therewith. One outlet of the dewatering unit 12 is connected with the inlet of the prepolymerization reactor 14 by line 20, whereas the prepolymerization reactor 14 is further connected via the return line 22 with the dewatering unit 12 and via line 24 with the inlet of lactide synthesis reactor 16. Moreover, the dewatering unit 12 comprises a water outlet line 25. In turn, the lactide synthesis reactor 16 is connected via the return line 26 with the prepolymerization reactor 14 and further comprises a purge line 28. Downstream of the lactide synthesis reactor 16, two further distillation columns 30, 32, namely a second distillation column 30 as well as a third distillation column 32, are provided. While the outlet of the lactide synthesis reactor 16 is connected with the inlet of the second distillation column 30 via line 34, the second distillation column 30 is connected via a return line 36 with the prepolymerization reactor 14, via a return line 38 with the lactide synthesis reactor 16 and via line 40 with the third distillation column 32. The third distillation column 32 comprises at its overhead a line 42 being connected with a static crystallizer 44, at its side a line 46 being connected with a falling film crystallizer 48 and at its bottom a return line 50, which is connected with the second distillation column 30. The static crystallizer 44 comprises an outlet line 52, which splits into the product line 54 and the connection line 56. In addition, the static crystallizer 44 comprises a purge line 58. In turn, the falling film crystallizer 48 comprises a first outlet line 60, which splits into the product line 62 and the connection line 64, as well as a second outlet line 66, which splits into a purge line 68 and into a return line 70, which leads into the second distillation column 30. Connection lines 56 and 64 combine into the monomer inlet line 72, which leads into the polylactic acid reactor 74. Also, an inlet line 76 for catalyst and initiator leads into the polylactic acid reactor 74. The polylactic acid reactor 74 further comprises an outlet line 78, which leads into a first devolatilizer 80. The first devolatilizer 80 comprises a vapor outlet line 82 and a melt outlet line 84 leading into a second devolatilizer 86, which comprises a vapor outlet line 88 and a polylactic acid outlet line 90. Furthermore, in plant 10 recycle lines 98, 98' and 98" are provided. More specifically, from the vapor outlet line 82 of the first devolatilizer 80 a recycle line 98 branches off, which in turn splits into recycle lines 98, 98' and 98". While recycle line 98' leads into the falling film crystallizer 48, the recycle line 98" leads into the static crystallizer 44.

As shown in detail in figure 2, the lactide synthesis reactor 16 comprises a first distillation column 104 and a loop reactor 106 being connected with the first distillation column 104. The first distillation column 104 is connected with the line 24 being connected with the outlet of the prepolymerization reactor 14. Moreover, the first distillation column 104 is connected with an overhead outlet line 108, with a side outlet line 34 and with a bottom outlet line 112. The overhead outlet line 108 is connected with a condenser 114, from which a recycle line 116 leads back into the first distillation column 104 and the return line 26 leads back into the prepolymerization reactor 14. Furthermore, the first distillation column 104 comprises three beds of structured packings 118, 118', 118", wherein the side outlet line 34 is connected with the first distillation column 104 at a position being arranged between, seen from the top to the bottom of the first distillation column 104, between the first and second beds of structured packings 118, 118'. While the bottom outlet line 112 of the first distillation column 104 is connected with an inlet 120 being arranged at a first position of the loop reactor 108, the loop reactor 108 comprises an outlet 122 being arranged at a second position of the loop reactor 108 and being connected with the first distillation column 104 via line 124. In addition, the loop reactor 106 comprises an inlet line 126 for catalyst, a heatable static mixer 128 and a purge outlet line 28.

During the operation of the plant 10, lactic acid is continuously fed via the inlet line 18 into the dewatering unit 12, in which the lactic acid is dewatered. Dewatered lactic acid is led via line 20 into the prepolymerization reactor 14, in which the lactic acid is prepolymerized so as to produce a lactic acid prepolymer, whereas the water having been separated from the lactic acid in the dewatering unit 12 is withdrawn therefrom through line 25. The lactic acid prepolymer is led via line 24 into the lactide synthesis reactor 16, in which the lactic acid prepolymer is depolymerized into a lactide mixture, which typically comprises meso-lactide, L-lactide and D-lactide. More specifically, the lactic acid prepolymer is fed via line 24 into the lower part of the first distillation column 14, from which it is led or pumped, respectively, via the inlet 120 into the loop reactor 106. Therein, the lactic acid prepolymer is converted in high yield to lactide, since the loop reactor 106 allows, not least due to the contained heatable static mixer 128, to adjust a homogenous reaction mixture, a sufficiently high and homogeneous catalyst concentration, an uniform temperature and an uniform pressure and thus to perform the depolymerization of the polylactic prepolymer to lactide at an optimal homogenous catalyst concentration, temperature and pressure. The so obtained lactide containing reaction composition is led via line 124 from the loop reactor 106 into the first distillation column 104, in which it is separated by distillation into an overhead composition including lactic acid, water and the like being withdrawn from the first distillation column 104 via the overhead outlet line 108, into a crude lactide composition being withdrawn from the first distillation column 104 via the side outlet line 34 and into non-reacted lactic acid prepolymer, which is recycled together with the lactic acid prepolymer composition being fed into the first distillation column 14 via the line 24 into the loop reactor 106 via the line 122 and via the inlet 120. While the overhead composition containing residual lactic acid is condensed in the condenser 114, from which a portion of the condensed overhead composition is recycled via the recycle line 116 into the first distillation column 104, the remaining portion of the condensed overhead composition is recycled via line 26 into the prepolymerization reactor 14. In addition, a purge stream is withdrawn from the loop reactor 106 of the lactide synthesis reactor 16 via line 28 and the crude lactide composition is led via line 34 from the lactide synthesis reactor 16 into the second distillation column 30. In the second distillation column 30, lights are separated from the lactides as overhead composition and are returned via line 36 into the prepolymerization reactor 14, whereas remaining lactic acid prepolymers are returned as bottom composition via line 38 into the lactide synthesis reactor 16 and the pre-purified lactide composition is led as side composition of the second distillation column 30 via line 40 into the third distillation column 32. The pre-purified lactide composition is separated in the third distillation column 32 into an overhead composition, into a side composition and into a bottom composition, wherein the overhead composition is the meso-lactide enriched composition and the side composition is the L-lactide enriched composition. While the meso-lactide enriched composition is fed via line 42 into the static crystallizer 44, the L-lactide enriched composition is fed via line 46 into the falling film crystallizer 48. Moreover, unreacted lactide from the first devolatilizer 80 is re-used in the polymerization step conducted in the polylactic acid reactor 74. Via the recycle lines 98, 98' and 98" portions of the vapor composition being withdrawn from the first devolatilizer 80 via line 82 are recycled into the static crystallizer 44 and into the falling film crystallizer 48. Purified meso-lactide enriched composition is withdrawn from the static crystallizer 44 via line 52, whereas the residue is withdrawn via purge line 58 as purge stream. Likewise thereto, purified L-lactide enriched composition is withdrawn from the falling film crystallizer 48 via line 60, whereas the residue is withdrawn via line 66 and is split into a recycle stream being fed into the third distillation column 32 via return line 70 and into a purge stream being withdrawn via purge line 68. The purified meso-lactide enriched composition is split into a portion being withdrawn as purified meso-lactide from the plant 10 via product line 54 as well as into a portion being fed via lines 56 and 72 into the polylactic acid reactor 74, whereas the purified L-lactide enriched composition is split into a portion being withdrawn as purified L-lactide from the plant 10 via product line 62 as well as into a portion being fed via lines 56 and 72 into the polylactic acid reactor 74. The lactide is polymerized in the polylactic acid reactor 74 in the presence of catalyst and initiator, which is fed into the polylactic acid reactor 74 via inlet line 76, to polylactic acid. The crude polylactic acid composition is fed via line 78 into a first devolatilizer 80, in which it is separated into a polylactic melt composition as well as into a vapor composition comprising unreacted lactide as well as traces of catalyst and initiator. While the vapor fraction is withdrawn from the first devolatilizer 80 via line 82, the polylactic melt composition is fed via line 84 into the second devolatilizer 86, in which it is separated into a vapor composition comprising unreacted lactide as well as traces of catalyst and initiator being withdrawn from the second devolatilizer 86 via line 88 as well as into purified polylactic acid, which is withdrawn from the plant 10 via line 90.

### Reference numerals

- 10: Plant
- 12: Dewatering unit
- 14: Prepolymerization reactor
- 16: Lactide synthesis reactor
- 18: Inlet line for lactic acid
- 20: Line
- 22: Return line
- 24: Line
- 25: Water outlet line
- 26: Return line
- 28: Purge line
- 30: (Second) distillation column
- 32: (Third) distillation column
- 34: Side outlet line for crude lactide composition
- 36: Return line
- 38: Return line
- 40: Line
- 42: Line
- 44: Static crystallizer
- 46: Line
- 48: Falling film crystallizer
- 50: Return line
- 52: Outlet line
- 54: Product line
- 56: Connection line
- 58, 58': Purge line
- 60: Outlet line
- 62: Product line
- 64: Connection line
- 66: Outlet line
- 68: Purge line
- 70: Return line
- 72: Monomer inlet line
- 74: Polylactic acid reactor
- 76: Inlet line
- 78: Outlet line
- 80: First devolatilizer
- 82: Vapor outlet line
- 84: Melt outlet line
- 86: Second devolatilizer
- 88: Vapor outlet line
- 90: Polylactic acid outlet line
- 98, 98', 98": Recycle line from devolatilization
- 104: (First) distillation column
- 106: Loop reactor
- 108: Overhead outlet line
- 112: Bottom outlet line
- 114: Condenser
- 116: Recycle line
- 118, 118', 118": Bed of structured packings
- 120: Inlet of loop reactor
- 122: Outlet of loop reactor
- 124: Line
- 126: Inlet line for catalyst
- 128: Heatable static mixer

## Claims

1. A method for preparing a crude lactide composition containing L-lactide and meso-lactide comprising the steps of subjecting a lactic acid composition to a prepolymerization reaction in a prepolymerization reactor so as to obtain a prepolymerized composition and then subjecting the prepolymerized composition to a depolymerization reaction in a lactide synthesis reactor, which comprises a distillation column and a loop reactor being connected therewith, so as to obtain the crude lactide composition, wherein the prepolymerized composition is fed into the distillation column, from which an overhead composition, a side composition as the crude lactide composition and a bottom composition are withdrawn, wherein the bottom composition is led into the loop reactor at a first position, wherein reaction product is withdrawn from the loop reactor at a second position and is led into the distillation column.

2. The method in accordance with claim 1, wherein the prepolymerization reaction is performed at a temperature of up to 190°C.

3. The method in accordance with claim 1 or 2, wherein the loop rector is heated, preferably using a heat exchanger, to a temperature of 190 to 240°C and preferably of 195 to 197°C.

4. The method in accordance with any of the preceding claims, wherein the loop rector comprises at least one static mixer being preferably selected from the group consisting of x-type static mixers, spiral/helical-type static mixers, quattro-type static mixers, baffle plate-type static mixers, turbulator strips-type static mixers and any combination of two or more of the above mentioned mixer types.

5. The method in accordance with any of the preceding claims, wherein the residence time in the loop reactor is below 8 hours and preferably below 6 hours.

6. The method in accordance with any of the preceding claims, wherein the distillation column comprises at least one, preferably one to ten, more preferably two to five, still more preferably two to four and most preferably three beds of an internal being selected from structured packings, random packings, plates, pipe bundles and arbitrary combinations of two or more thereof.

7. The method in accordance with any of the preceding claims, wherein the distillation column is operated at a temperature of below 230°C and a pressure of below 1 kPa and preferably at a temperature of below 210°C and a pressure of below 0.5 kPa.

8. The method in accordance with any of the preceding claims, wherein the overhead composition is led into a condenser and is condensed therein, wherein a portion of the so obtained condensate is withdrawn and preferably recycled into the prepolymerization reactor, whereas the remaining portion thereof is recycled into the distillation column.

9. The method in accordance with any of the preceding claims, wherein the crude lactide composition comprises, based on 100% by weight of the crude lactide composition, at least 85% by weight and preferably at least 92% by weight of lactide.

10. A method for preparing a purified L-lactide composition and optionally also a purified meso-lactide composition comprising the following steps:
a) preparing a crude lactide composition containing L-lactide and meso-lactide with a method in accordance with any of the preceding claims,
b) feeding the crude lactide composition obtained in step a) into a second distillation column and distilling it therein into an overhead composition, into a bottom composition and into a side composition and
c) feeding the side composition obtained in step b) into a third distillation column and distilling it therein into an overhead composition being a meso-lactide enriched composition, a side composition being a L-lactide enriched composition and a bottom composition.

11. The method in accordance with claim 10, wherein the L-lactide enriched composition obtained in step c) is further purified by subjecting it to at least one desublimation step and/or to at least one falling film crystallization step, and wherein the meso-lactide enriched composition obtained in step c) is further purified by subjecting it to at least one desublimation step and/or to at least one static crystallization step.

12. A plant for preparing a crude lactide composition containing L-lactide and meso-lactide comprising:
i) at least one prepolymerization reactor comprising an inlet and an outlet, and
ii) a lactide synthesis reactor comprising a distillation column and a loop reactor being connected therewith, wherein the distillation column comprises an inlet being connected with the outlet of the prepolymerization reactor, an overhead outlet, a side outlet and a bottom outlet, wherein the bottom outlet is connected with an inlet being arranged at a first position of the loop reactor, and wherein the loop reactor comprises an outlet being arranged at a second position and being connected with the distillation column.

13. The plant in accordance with claim 12, wherein the loop reactor further comprises an inlet for catalyst, an outlet for purge, at least one heat exchanger and at least one static mixer being preferably selected from the group consisting of x-type static mixers, spiral/helical-type static mixers, quattro-type static mixers, baffle plate-type static mixers, turbulator strips-type static mixers and any combination of two or more of the above mentioned mixer types.

14. A plant for preparing a purified L-lactide composition and optionally also a purified meso-lactide composition comprising:
a) a plant in accordance with claim 12 or 13,
b) a second distillation column comprising an inlet for crude composition being connected with the side outlet of the distillation column of the lactide synthesis reactor, an overhead outlet, a bottom outlet and a side outlet, and
ii) a third distillation column comprising an inlet being connected with the side outlet of the second distillation column, wherein the third distillation column further comprises an overhead outlet, a bottom outlet and a side outlet.

15. The plant in accordance with claim 14, which further comprises a recycle line connecting the overhead outlet of the second distillation column with an inlet of the prepolymerization reactor, a recycle line connecting the bottom outlet of the second distillation column with an inlet of the distillation column of the lactide synthesis reactor, and a recycle line connecting the bottom outlet of the third distillation column with an inlet of the second distillation column.
